# NOUVEAU FASCICULE DE BREVET EUROPEEN

(11) **EP 1 047 383 B2**
(45) Date de publication et mention de la décision concernant l'opposition: **17.12.2008**
(45) Mention de la délivrance du brevet: 09.04.2003
(21) Numéro de dépôt: 98963598.2
(22) Date de dépôt: 22.12.1998
(51) Int. Cl.: A61K 8/41, A61K 8/34, A61Q 5/10

(54) **COMPOSITION DE TEINTURE D'OXYDATION DES FIBRES KERATINIQUES CONTENANT UNE LACCASE ET PROCEDE DE TEINTURE METTANT EN OEUVRE CETTE COMPOSITION**
OXIDATIONFÄRBEMITTEL FÜR KERATINFASERN, DAS EINE LACCASE ENTHÄLT, UND VERFAHREN ZUR FÄRBUNG MIT DIESEM MITTEL
KERATINOUS FIBRE OXIDATION DYEING COMPOSITION CONTAINING A LACCASE AND DYEING METHOD USING SAME

(30) Priorité: 13.01.1998 FR 9800257
(43) Date de publication de la demande: 02.11.2000
(73) Titulaire: L'ORÉAL, 75008 Paris (FR)
(72) Inventeur: LANG, Gérard, F-95390 Saint Prix (FR); COTTERET, Jean, F-78480 Verneuil sur Seine (FR)
(74) Mandataire: Dossmann, Gérard
(86) Numéro de dépôt international: PCT/FR1998/002833
(87) Numéro de publication internationale: WO 1999/036041

(56) Documents cités:
- EP-A- 0 504 005
- EP-A- 0 634 163
- EP-A- 0 667 143
- WO-A1-95/33836
- WO-A1-96/00290
- WO-A1-97/19998
- WO-A1-97/19999
- WO-A1-97/37633
- WO-A1-98/22078
- DE-A- 2 155 359
- DE-A1- 4 341 998
- DK-A- 35 898
- DK-A- 151 397
- FR-A- 2 694 018
- US-A- 3 251 742
- US-A- 4 420 637
- US-A- 4 904 275

## Description

L'invention a pour objet une composition pour la teinture d'oxydation des fibres kératiniques, et en particulier des fibres kératiniques humaines telles que les cheveux comprenant, dans un milieu approprié pour la teinture, au moins une première base d'oxydation choisie parmi les paraphénylènediamines, au moins une seconde base d'oxydation choisie parmi les para-aminophénols, au moins un coupleur choisi parmi les méta-aminophénols, les méta-phénylènediamines et les méta-diphénols, et au moins une enzyme de type laccase, ainsi que le procédé de teinture mettant en oeuvre cette composition.

Il est connu de teindre les fibres kératiniques et en particulier les cheveux humains avec des compositions tinctoriales contenant des précurseurs pour la coloration d'oxydation, en particulier des ortho ou paraphénylènediamines, des ortho ou paraaminophénols, des bases hétérocycliques, appelés généralement bases d'oxydation. Les précurseurs pour la coloration d'oxydation, (bases d'oxydation), sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présentent pour inconvénient d'entraîner une dégradation non négligeable des fibres, ainsi qu'une décoloration des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé dans le brevet US 3 251 742, et les demandes de brevet FR-A-2 112 549, FR-A-2 694 018, EP-A-0 504 005, WO95/07988, WO95/33836, WO95/33837, WO96/00290, WO97/19998 et WO97/19999 de teindre les fibres kératiniques avec des compositions comprenant au moins un colorant d'oxydation, ou au moins un précurseur de mélanine, en association avec des enzymes du type laccase ; lesdites compositions étant mises en contact avec l'oxygène de l'air. Ces formulations de teinture, bien qu'étant mises en oeuvre dans des conditions n'entraînant pas une dégradation des fibres kératiniques comparable à celle engendrée par les teintures réalisées en présence de peroxyde d'hydrogène, conduisent à des colorations encore insuffisantes à la fois sur le plan de l'homogénéité de la couleur répartie le long de la fibre (unisson), sur le plan de la chromaticité (luminosité) et de la puissance tinctoriale.

Or, la demanderesse vient maintenant de découvrir qu'il est possible d'obtenir de nouvelles teintures, capables de conduire à des colorations puissantes sans engendrer de dégradation significative des fibres kératiniques, peu sélectives et résistant bien aux diverses agressions que peuvent subir les fibres, en associant au moins première base d'oxydation choisie parmi les paraphénylènediamines, au moins une seconde base d'oxydation choisie parmi les para-aminophénols, au moins un coupleur choisi parmi les méta-aminophénols, les méta-phénylènediamines et les méta-diphénols, et au moins une enzyme de type laccase.

Cette découverte est à la base de la présente invention.

L'invention a donc pour premier objet une composition prête à l'emploi, pour la teinture d'oxydation des fibres kératiniques et en particulier des fibres kératiniques humaines telles que les cheveux, caractérisée par le fait qu'elle comprend, dans un milieu approprié pour la teinture :
- au moins une première base d'oxydation choisie parmi les paraphénylènediamines,
- au moins une seconde base d'oxydation choisie parmi les para-aminophénols,
- au moins un coupleur choisi parmi les méta-aminophénols, les méta-phénylènediamines et les méta-diphénols, et
- au moins une enzyme de type laccase ;
ladite composition comprenant la para-phénylènediamine, le para-aminophénol et le 2-méthyl-5-aminophénol;
ou comprenant la para-phénylènediamine, le para-aminophénol et le 2,4-diamino-1-(β-hydroxyéthyloxy)benzène;
ou comprenant la para-phénylènediamine, le para-aminophénol et le 1,3 dihydroxybenzène,
étant entendu que :
- lorsque ladite composition contient de la paraphénylènediamine ou l'un de ses sels d'addition avec un acide à titre de première base d'oxydation et du para-aminophénol à titre de seconde base d'oxydation, alors le coupleur ne peut pas être choisi parmi le 4-chloro résorcinol, la méta-phénylènediamine, et ses sels d'addition avec un acide ; et
- lorsque ladite composition contient de la paratoluylènediamine à titre de première base d'oxydation et du para-aminophénol à titre de seconde base d'oxydation, alors le coupleur ne peut pas être choisi parmi le 5-amino 2-méthyl phénol et la résorcine.

La composition tinctoriale prête à l'emploi conforme à l'invention conduit à des colorations puissantes présentant une faible sélectivité et d'excellentes propriétés de résistances à la fois vis à vis des agents atmosphériques tels que la lumière et les intempéries et vis à vis de la transpiration et des différents traitements que peuvent subir les cheveux (lavages, déformations permanentes).

L'invention a également pour objet un procédé de teinture d'oxydation des fibres kératiniques mettant en oeuvre cette composition tinctoriale prête à l'emploi.

La ou les laccases utilisées dans la composition tinctoriale prête à l'emploi conforme à l'invention peuvent notamment être choisies parmi les laccases d'origine végétale, d'origine animale, d'origine fongique (levures, moisissures, champignons) ou d'origine bactérienne, les organismes d'origine pouvant être mono- ou pluricellulaires. La ou les laccases utilisées dans la composition tinctoriale prête à l'emploi conforme à l'invention peuvent également être obtenues par biotechnologie.

Parmi les laccases d'origine végétale utilisables selon l'invention, on peut citer les laccases produites par des végétaux effectuant la synthèse chlorophyllienne telles que celles indiquées dans la demande de brevet FR-A-2 694 018.

On peut notamment citer les laccases présentes dans les extraits d'Anacardiacées tels que par exemple les extraits de Magnifera indica, de Schinus molle ou de Pleiogynium timoriense ; dans les extraits de Podocarpacées ; de Rosmarinus off. ; de Solanum tuberosum ; d'Iris sp. ; de Coffea sp. ; de Daucus carrota ; de Vinca minor ; de Persea americana ; de Catharenthus roseus ; de Musa sp. ; de Malus pumila ; de Gingko biloba ; de Monotropa hypopithys (sucepin), d'Aesculus sp. ; d'Acer pseudoplatanus ; de Prunus persica et de Pistacia palaestina.

Parmi les laccases d'origine fongique, éventuellement obtenues par biotechnologie, utilisables selon l'invention, on peut citer la ou les laccases issues de Polyporus versicolor, de Rhizoctonia praticola et de Rhus vernicifera telles que décrites par exemples dans les demandes de brevet FR-A-2 112 549 et EP-A-504005 ; les laccases décrites dans les demandes de brevet WO95/07988, WO95/33836, WO95/33837, WO96/00290, WO97/19998 et WO97/19999, dont le contenu fait partie intégrante de la présente description comme par exemple la ou les laccases issues de Scytalidium, de Polyporus pinsitus, de Myceliophtora thermophila, de Rhizoctonia solani, de Pyricularia orizae, et leurs variantes. On peut également citer la ou les laccases-issues de Trametes versicolor, de Fomes fomentarius, de Chaetomium thermophile, de Neurospora crassa, de Colorius versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, et de leurs variantes.

On choisira plus préférentiellement les laccases d'origine fongiques, éventuellement obtenues par biotechnologie.

L'activité enzymatique des laccases utilisées conformément à l'invention et ayant la syringaldazine parmi leurs substrat peut être définie à partir de l'oxydation de la syringaldazine en condition aérobie. L'unité Lacu correspond à la quantité d'enzyme catalysant la conversion de 1 mmole de syringaldazine par minute à un pH de 5,5 et à une température de 30°C. L'unité U correspond à la quantité d'enzyme produisant un delta d'absorbance de 0,001 par minute, à une longueur d'onde de 530 nm, en utilisant la syringaldazine comme substrat, à 30°C et à un pH de 6,5. L'activité enzymatique des laccases de l'invention peut aussi être définie à partir de l'oxydation de la paraphénylènediamine. L'unité ulac correspond à la quantité d'enzyme produisant un delta d'absorbance de 0,001 par minute, à une longueur d'onde de 496,5 nm, en utilisant la paraphénylènediamine comme substrat (64 mM), à 30°C et à un pH de 5.

Selon l'invention, on préfère déterminer l'activité enzymatique en unités ulac.

La quantité de laccase(s) présente dans la composition tinctoriale prête à l'emploi conforme à l'invention variera en fonction de la nature de la ou des laccases utilisées. De façon préférentielle, la quantité de laccase(s) est comprise entre 0,5 et 200 Lacu environ (soit entre 10000 et 4.10⁶ unités U environ ou soit entre 20 et 2.10⁶ unités ulac) pour 100 g de composition tinctoriale.

Parmi les paraphénylènediamines utilisables dans la composition tinctoriale prête à l'emploi conforme à l'invention, on peut notamment citer les composés de formule (I) suivante, et leurs sels d'addition avec un acide : 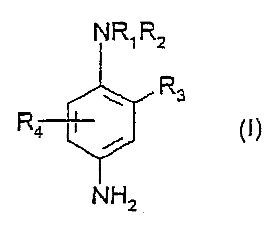 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄.

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (I) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxy-éthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines de formule (I) ci-dessus, on préfère tout particulièrement la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

La ou les paraphénylènediamines utilisées représentent de préférence de 0,0005 à 12 % en poids environ du poids total de la composition tinctoriale prête à l'emploi conforme à l'invention, et encore plus préférentiellement de 0,005 à 6 % en poids environ de ce poids.

Parmi les para-aminophénols utilisables dans les compositions tinctoriales conformes à l'invention, on peut notamment citer les composés répondant à la formule (II) suivante, et leurs sels d'addition avec un acide : 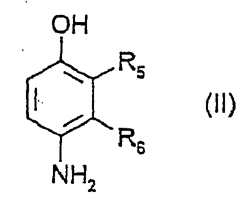 dans laquelle :
- R₅ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₆ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₅ ou R₆ représente un atome d'hydrogène.

Parmi les para-aminophénols de formule (II) ci-dessus, on peut plus particulièrement citer le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Le ou les para-aminophénols utilisés représentent de préférence de 0,0005 à 12 % en poids environ du poids total de la composition tinctoriale prête à l'emploi conforme à l'invention, et encore plus préférentiellement de 0,005 à 6 % en poids environ de ce poids.

Le ou les coupleurs utilisés conformément à l'invention, c'est à dire le ou les méta-phénylènediamines et/ou le ou les méta-aminophénols et/ou le ou les méta-diphénols représentent de préférence de 0,0001 à 10 % en poids environ du poids total de la composition tinctoriale prête à l'emploi, et encore plus préférentiellement de 0,005 à 5 % en poids environ de ce poids.

La composition tinctoriale prête à l'emploi conforme à l'invention peut en outre contenir un ou plusieurs coupleurs additionnels différents des coupleurs mentionnés précédemment et/ou un ou plusieurs colorants directs, notamment pour modifier les nuances ou les enrichir en reflets.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau.

Le pH de la composition prête à l'emploi conforme à l'invention est choisi de telle manière que l'activité enzymatique de la laccase soit suffisante. Il est généralement compris entre 4 et 11 environ, et de préférence entre 6 et 9 environ. Il peut être ajusté à la valeur désirée au moyen d'agents acidifiants ou alcalinisants habituellement utilisés en teinture des fibres kératiniques.

La composition tinctoriale prête à l'emploi conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères, des agents antioxydants, des enzymes différentes des laccases utilisées conformément à l'invention telles que par exemples des peroxydases ou des oxydo-réductases à 2 électrons, des agents de pénétration, des agents séquestrants, des parfums, des tampons, des agents dispersants, des agents épaississants, des agents filmogènes, des agents conservateurs, des agents opacifiants, des vitamines.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains. Dans ce cas, les colorants d'oxydation et la ou les enzymes de type laccase sont présents au sein de la même composition prête à l'emploi, et par conséquent ladite composition doit être exempte d'oxygène gazeux, de manière à éviter toute oxydation prématurée des colorants d'oxydation.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Les exemples qui suivent sont destinés à illustrer l'invention sans pour autant en limiter la portée.

### EXEMPLES

### EXEMPLES 1 à 3 DE TEINTURE

On a préparé les compositions tinctoriales prêtes à l'emploi suivantes (teneurs en grammes) :

| **COMPOSITION** | **1** | **2** | **3** |
|---|---|---|---|
| Paraphénylènediamine (première base d'oxydation) | 0,17 | 0,17 | 0,17 |
| Para-aminophénol (seconde base d'oxydation) | 0,08 | 0,08 | 0,08 |
| 2-méthyl 5-amino phénol (coupleur) | 0,14 | - | - |
| Dichlorhydrate de 2,4-diamino 1-(β-hydroxyéthyloxy) benzène (coupleur) | - | 0,14 | - |
| 1,3-dihydroxy benzène (coupleur) | - | - | 0,12 |
| Laccase issue de Rhus vernicifera à 180 unités / mg vendue par la société Sigma | 1,8 | 1,8 | 1,8 |
| Support de teinture commun (*) | (*) | (*) | (*) |
| Eau déminéralisée q.s.p. | 100 g | 100 g | 100 g |

| | | | |
|---|---|---|---|
| (*) : Support de teinture commun : - Alkyl (C₈-C₁₀) polyglucoside en solution aqueuse à 60 % de matière active (M.A.), vendu sous la dénomination ORAMIX CG110 ® par la société SEPPIC 8,0 g - Ethanol 20 g - Agent de pH q.s. pH 6,5 | | | |

Chacune des compositions tinctoriales prêtes à l'emploi décrites ci-dessus a été appliquée sur des mèches de cheveux gris naturels à 90 % de blancs pendant 40 minutes, et à une température de 30°C. Les cheveux ont ensuite été rincés, puis séchés.

Les cheveux ont été teints dans les nuances figurant dans le tableau ci-après :

| **EXEMPLE** | **Nuance** **obtenue** |
|---|---|
| **1** | Blond acajou |
| **2** | Blond violine |
| **3** | Blond doré |

Dans les compositions tinctoriales décrites ci-dessus, la laccase de Rhus vemicifera à 180 unités / mg, vendue par la société Sigma peut être remplacée par 1,0 g de laccase de Pyricularia orizae à 100 unités / mg vendue par la société ICN.

## Revendications

1. Composition prête à l'emploi, pour la teinture d'oxydation des fibres kératiniques et en particulier des fibres kératiniques humaines telles que les cheveux, **caractérisée par le fait qu**'elle comprend, dans un milieu approprié pour la teinture :
- au moins une première base d'oxydation choisie parmi les paraphénylènediamines,
- au moins une seconde base d'oxydation choisie parmi les para-aminophénols,
- au moins un coupleur choisi parmi les méta-aminophénols, les méta-phénylènediamines et les méta-diphénols, et
- au moins une enzyme de type laccase ;
ladite composition comprenant la para-phénylènediamine, le para-aminophénol et le 2-méthyl-5-aminophénol;
ou comprenant la para-phénylènediamine, le para-aminophénol et le 2,4-diamino-1-(β-hydroxyéthyloxy)benzène;
ou comprenant la para-phénylènediamine, le para-aminophénol et le 1,3 dihydroxybenzène,
étant entendu que :
- lorsque ladite composition contient de la paraphénylènediamine ou l'un de ses sels d'addition avec un acide à titre de première base d'oxydation et du para-aminophénol à titre de seconde base d'oxydation, alors le coupleur ne peut pas être choisi parmi le 4-chloro résorcinol, la méta-phénylènediamine, et ses sels d'addition avec un acide ; et
- lorsque ladite composition contient de la paratoluylènediamine à titre de première base d'oxydation et du para-aminophénol à titre de seconde base d'oxydation, alors le coupleur ne peut pas être choisi parmi le 5-amino 2-méthyl phénol et la résorcine.

2. Composition selon la revendication 1, **caractérisée par le fait que** la laccase est choisie parmi les laccases d'origine végétale, d'origine animale, d'origine fongique ou d'origine bactérienne et parmi les laccases obtenues par biotechnologie.

3. Composition selon l'une quelconque des revendications 1 ou 2, **caractérisée par le fait que** la laccase est d'origine végétale et choisie parmi les laccases présentes dans les extraits d'Anacardiacées ; de Podocarpacées ; de Rosmarinus off. ; de Solanum tuberosum ; d'Iris sp. ; de Coffea sp. ; de Daucus carrota ; de Vinca minor ; de Persea americana ; de Catharenthus roseus ; de Musa sp. ; de Malus pumila ; de Gingko biloba ; de Monotropa hypopithys (sucepin), d'Aesculus sp. ; d'Acer pseudoplatanus ; de Prunus persica et de Pistacia palaestina.

4. Composition selon la revendication 1 ou 2, **caractérisée par le fait que** la laccase est d'origine microbienne ou obtenue par biotechnologie.

5. Composition selon la revendication 4, **caractérisée par le fait que** la laccase est choisie parmi les laccases issues de Polyporus versicolor, de Rhizoctonia praticola, de Rhus vernicifera, de Scytalidium, de Polyporus pinsitus, de Myceliophtora thermophila, de Rhizoctonia solani, de Pyricularia orizae, de Trametes versicolor, de Fomes fomentarius, de Chaetomium thermophile, de Neurospora crassa, de Colorius versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius-piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, et de leurs variantes.

6. Composition selon l'une quelconque des revendications précédentes, **caractérisée par le fait que** la quantité de laccase(s) est comprise entre 0,5 et 200 Lacu pour 100 g de composition tinctoriale.

7. Composition selon l'une quelconque des revendications précédentes, **caractérisée par le fait que** la paraphénylènediamine représente de 0,0005 à 12 % en poids du poids total de la composition tinctoriale prête à l'emploi.

8. Composition selon la revendication 7, **caractérisée par le fait qu**e la paraphénylènediamine représente de 0,005 à 6 % en poids du poids total de la composition tinctoriale prête à l'emploi.

9. Composition selon l'une quelconque des revendications précédentes, **caractérisée par le fait que** le ou les para-aminophénols représentent de 0,0005 à 12 % en poids du poids total de la composition tinctoriale prête à l'emploi.

10. Composition selon la revendication 9, **caractérisée par le fait que** le ou les para-aminophénols représentent de 0,005 à 6 % en poids du poids total de la composition tinctoriale prête à l'emploi.

11. Composition selon l'une quelconque des revendications précédentes, **caractérisée par le fait que** le ou les méta-phénylènediamines et/ou le ou les méta-aminophénols et/ou le ou les méta-diphénols représentent de 0,0001 à 10 % en poids du poids total de la composition tinctoriale prête à l'emploi.

12. Composition selon la revendication 11, **caractérisée par le fait que** le ou les méta-phénylènediamines et/ou le ou les méta-aminophénols et/ou le ou les méta-diphénols représentent de 0,005 à 5 % en poids du poids total de la composition tinctoriale prête à l'emploi.

13. Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétate.

14. Composition selon l'une quelconque des revendications précédentes, **caractérisée par le fait que** le milieu approprié pour la teinture est constitué par de l'eau ou par un mélange d'eau et d'au moins un solvant organique.

15. Composition selon l'une quelconque des revendications précédentes, **caractérisée par le fait qu'**elle présente un pH compris 4 et 11.

16. Procédé de teinture des fibres kératiniques et en particulier des fibres kératiniques humaines telles que les cheveux, **caractérisé par le fait qu**'on applique sur lesdites fibres au moins une composition tinctoriale prête à l'emploi telle que définie dans l'une quelconque des revendications précédentes, pendant un temps suffisant pour développer la coloration désirée.

## Claims

1. Ready-to-use composition for the oxidation dyeing of keratinous fibres, and in particular human keratinous fibres such as hair, **characterized in that** it comprises, in a medium appropriate for dyeing:
- at least one first oxidation base chosen from para-phenylenediamines,
- at least one second oxidation base chosen from para-aminophenols,
- at least one coupler chosen from meta-aminophenols, meta-phenylenediamines and meta-diphenols, and
- at least one enzyme of the laccase type;
the said composition comprising para-phenylenediamine, para-aminophenol and 2-methyl-5-aminophenol;
or comprising para-phenylenediamine, para-aminophenol and 2,4-diamino-1-(β-hydroxyethyloxy)benzene;
or comprising para-phenylenediamine, para-aminophenol and 1,3-dihydroxybenzene,
it being understood that:
- when the said composition contains para-phenylenediamine or one of its addition salts with an acid as first oxidation base and para-aminophenol as second oxidation base, then the coupler cannot be chosen from 4-chlororesorcinol, meta-phenylenediamine, and its addition salts with an acid; and
- when the said composition contains para-tolylenediamine as first oxidation base and para-aminophenol as second oxidation base, then the coupler cannot be chosen from 5-amino-2-methylphenol and resorcinol.

2. Composition according to Claim 1, **characterized in that** the laccase is chosen from laccases of plant origin, animal origin, fungal origin or bacterial origin and from laccases obtained by biotechnology.

3. Composition according to either of Claims 1 and 2, **characterized in that** the laccase is of plant origin and chosen from the laccases present in the extracts of Anacardiaceae, Podocarpaceae, Rosmarinus off., Solanum tuberosum, Iris sp., Coffea sp., Daucus carrota, Vinca minor, Persea americana, Catharenthus roseus, Musa sp., Malls pumila, Gingko biloba, Monotropa hypopithys (Indian pipe), Aesculus sp., Acer pseudoplatanus, Prunus persica and Pistacia palaestina.

4. Composition according to Claim 1 or 2, **characterized in that** the laccase is of microbial origin or obtained by biotechnology.

5. Composition according to Claim 4, **characterized in that** the laccase is chosen from the laccases derived from Polyporus versicolor, Rhizoctonia praticola, Rhus vernicifera, Scytalidium, Polyporus pinsitus, Myceliophtora thermophila, Rhizoctonia solani, Pyricularia orizae, Trametes versicolor, Fomes fomentarius, Chaetomium thermophile, Neurospora crassa, Colorius versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitius squalens, and variants thereof.

6. Composition according to any one of the preceding claims, **characterized in that** the quantity of laccase(s) is between 0.5 and 200 Lacu per 100 g of dyeing composition.

7. Composition according to any one of the preceding claims, **characterized in that** the para-phenylenediamine represents from 0.0005 to 12% by weight of the total weight of the ready-to-use dyeing composition.

8. Composition according to Claim 7, **characterized in that** the para-phenylenediamine represents from 0.005 to 6% by weight of the total weight of the ready-to-use dyeing composition.

9. Composition according to any one of the preceding claims, **characterized in that** the para-aminophenol(s) represent from 0.0005 to 12% by weight of the total weight of the ready-to-use dyeing composition.

10. Composition according to Claim 9, **characterized in that** the para-aminophenol(s) represent from 0.005 to 6% by weight of the total weight of the ready-to-use dyeing composition.

11. Composition according to any one of the preceding claims, **characterized in that** the meta-phenylenediamine(s) and/or the meta-aminophenol(s) and/or the meta-diphenol(s) represent from 0.0001 to 10% by weight of the total weight of the ready-to-use dyeing composition.

12. Composition according to Claim 11, **characterized in that** the meta-phenylenediamine(s) and/or the meta-aminophenol(s) and/or the meta-diphenol(s) represent from 0.005 to 5% by weight of the total weight of the ready-to-use dyeing composition.

13. Composition according to any one of the preceding claims, **characterized in that** the addition salts with an acid are chosen from hydrochlorides, hydrobromides, sulphates and tartrates, lactates and acetates.

14. Composition according to any one of the preceding claims, **characterized in that** the medium appropriate for dyeing consists of water or of a mixture of water and at least one organic solvent.

15. Composition according to any one of the preceding claims, **characterized in that** it has a pH of between 4 and 11.

16. Method of dyeing keratinous fibres, and in particular human keratinous fibres such as hair, **characterized in that** at least one ready-to-use dyeing composition as defined in any one of the preceding claims is applied to the said fibres for a sufficient time to develop the desired colour.

## Patentansprüche

1. Gebrauchsfertige Zusammensetzung zum oxidativen Färben von Keratinfasern und insbesondere menschlichen Keratinfasern, wie Haaren, **dadurch gekennzeichnet, dass** sie in einem zum Färben geeigneten Medium enthält:
- mindestens eine erste Oxidationsbase, die unter den p-Phenylendiaminen ausgewählt ist,
- mindestens eine zweite Oxidationsbase, die unter den p-Aminophenolen ausgewählt ist,
- mindestens einen Kuppler, der unter den m-Aminophenolen, m-Phenylendiaminen und m-Dihydroxybenzolen ausgewählt ist, und
- mindestens ein Enzym vom Laccase-Typ;
wobei die Zusammensetzung para-Phenylendiamin, para-Aminophenol und 2-Methyl-5-aminophenol; oder para-Phenylendiamin, para-Aminophenol und 2,4-Diamino-1-(β-hydroxyethyloxy)-benzol; oder para-Phenylendiamin, para-Aminophenol und 1,3-Dihydroxybenzol enthält; mit der Maßgabe, dass
- der Kuppler nicht unter 4-Chlorresorcin, m-Phenylendiamin und ihren Additionssalzen mit einer Säure ausgewählt werden kann, wenn die Zusammensetzung das p-Phenylendiamin oder eines seiner Additionssalze mit einer Säure als erste Oxidationsbase und das p-Aminophenol als zweite Oxidationsbase enthält; und
- der Kuppler nicht unter 5-Amino-2-methyl-phenol und Resorcin ausgewählt werden kann, wenn die Zusammensetzung das p-Toluylendiamin als erste Oxidationsbase und das p-Aminophenol als zweite Oxidationsbase enthält.

2. Zusammensetzung nach Anspruch 1, **dadurch gekennzeichnet, dass** die Laccase unter den Laccasen pflanzlicher Herkunft, den Laccasen tierischer Herkunft, den Laccasen, die von Pilzen gebildet werden, den Laccasen bakterieller Herkunft oder den biotechnologisch hergestellten Laccasen ausgewählt ist.

3. Zusammensetzung nach einem der Ansprüche 1 oder 2, **dadurch gekennzeichnet, dass** die Laccase pflanzlicher Herkunft ist und unter den Laccasen ausgewählt ist, die in Extrakten von Anacardiaceae, Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Fichtenspargel), Aesculus sp., Acer pseudoplatanus, Prunus persica oder Pistacia palaestina vorliegen.

4. Zusammensetzung nach Anspruch 1 oder 2, **dadurch gekennzeichnet, dass** die Laccase mikrobieller Herkunft oder biotechnologisch hergestellt ist.

5. Zusammensetzung nach Anspruch 4, **dadurch gekennzeichnet, dass** die Laccase unter den Laccasen ausgewählt ist, die von Polyporus versicolor, Rhizoctonia praticola, Rhus vernicifera, Scytalidium, Polyporus pinsitus, Myceliophtora thermophila, Rhizoctonia solani, Pyricularia orizae, Tramates versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitus squalens und deren Varianten stammen.

6. Zusammensetzung nach einem der vorhergehenden Ansprüche, **dadurch gekennzeichnet, dass** die Laccase(n) in einem Mengenanteil im Bereich von 0,5 bis 200 lacu pro 100 g Zusammensetzung vorliegen.

7. Zusammensetzung nach einem der vorhergehenden Ansprüche, **dadurch gekennzeichnet, dass** das p-Phenylendiamin 0,0005 bis 12 Gew.-% des Gesamtgewichts der gebrauchsfertigen Farbmittelzusammensetzung ausmacht.

8. Zusammensetzung nach Anspruch 7, **dadurch gekennzeichnet, dass** das p-Phenylendiamin 0,005 bis 6 Gew.-% des Gesamtgewichts der gebrauchsfertigen Farbmittelzusammensetzung ausmacht.

9. Zusammensetzung nach einem der vorhergehenden Ansprüche, **dadurch gekennzeichnet, dass** das p-Aminophenol oder die p-Aminophenole 0,0005 bis 12 Gew.-% des Gesamtgewichts der gebrauchsfertigen Farbmittelzusammensetzung ausmachen.

10. Zusammensetzung nach Anspruch 9, **dadurch gekennzeichnet, dass** das p-Aminophenol oder die p-Aminophenole 0,005 bis 6 Gew.-% des Gesamtgewichts der gebrauchsfertigen Farbmittelzusammensetzung ausmachen.

11. Zusammensetzung nach einem der vorhergehenden Ansprüche, **dadurch gekennzeichnet, dass** das m-Phenylendiamin oder die m-Phenylendiamine und/oder das m-Aminophenol oder die m-Aminophenole und/oder das m-Dihydroxybenzol oder die m-Dihydroxybenzole 0,0001 bis 10 Gew.-% des Gesamtgewichts der erfindungsgemäßen gebrauchsfertigen Farbmittelzusammensetzung ausmachen.

12. Zusammensetzung nach Anspruch 11, **dadurch gekennzeichnet, dass** das m-Phenylendiamin oder die m-Phenylendiamine und/oder das m-Aminophenol oder die m-Aminophenole und/oder das m-Dihydroxybenzol oder die m-Dihydroxybenzole 0,005 bis 5 Gew.-% des Gesamtgewichts der erfindungsgemäßen gebrauchsfertigen Farbmittelzusammensetzung ausmachen.

13. Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

14. Zusammensetzung nach einem der vorhergehenden Ansprüche, **dadurch gekennzeichnet, dass** das zum Färben geeignete Medium aus Wasser oder einem Gemisch von Wasser und mindestens einem organischen Lösungsmittel besteht.

15. Zusammensetzung nach einem der vorhergehenden Ansprüche, **dadurch gekennzeichnet, dass** sie einen pH-Wert im Bereich von 4 bis 11 aufweist.

16. Verfahren zum Färben von Keratinfasern und insbesondere menschlichen Keratinfasern, wie Haaren, **dadurch gekennzeichnet, dass** auf die Fasern mindestens eine gebrauchsfertige Farbmittelzusammensetzung nach einem der vorhergehenden Ansprüche während einer Zeitspanne, die zur Bildung der gewünschten Färbung ausreichend ist, aufgebracht wird.
